(19) Europäisches Patentamt
European Patent Office
Office européen des brevets

(11) EP 2 065 058 A1

(12) EUROPEAN PATENT APPLICATION

(43) Date of publication:
**03.06.2009 Bulletin 2009/23**

(51) Int Cl.:
***A61K 49/18*** *(2006.01)*

(21) Application number: **07121809.3**

(22) Date of filing: **28.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Koninklijke Philips Electronics N.V.**
**5621 BA Eindhoven (NL)**

(72) Inventor: **The designation of the inventor has not yet been filed**

(74) Representative: **Van Velzen, Maaike Mathilde**
**Philips**
**Intellectual Property & Standards**
**P.O. Box 220**
**5600 AE Eindhoven (NL)**

# (54) Non-spherical contrast agents for CEST MRI based on bulk magnetic susceptibility effect

(57) In magnetic resonance imaging (MRI) based on chemical exchange-dependent saturation transfer (CEST), a novel carrier for CEST contrast agents is provided. The carrier is non-spherical and comprises a semipermeable shell, wherein the shell comprises a paramagnetic compound. The shell encloses a cavity comprising an MR analyte, wherein the semipermeable shell allows diffusion of the MR analyte. The CEST effect is based on the bulk magnetic susceptibility effect caused by the anisotropy of the carrier. This leads to a versatile carrier that does not require interaction of the analyte with a paramgnetic chemical shift reagent.

EP 2 065 058 A1

## Description

FIELD OF THE INVENTION

The invention relates to Magnetic Resonance Imaging (MRI) based on Chemical Exchange-dependent Saturation Transfer (CEST). More particularly, the invention relates to CEST MRI contrast agents based on non-spherical carriers comprising a semipermeable shell, the shell enclosing a cavity comprising an MR analyte.

BACKGROUND OF THE INVENTION

Magnetic Resonance Imaging (MRI) is an important diagnostic technique that is commonly used in hospitals for the diagnosis of disease. MRI allows for the non-invasive imaging of soft tissue with a superb spatial resolution.

Almost all current MRI scans are based on the imaging of bulk water molecules that are present at a very high concentration throughout the whole body in all tissues. If the contrast between different tissues is insufficient to obtain clinical information, MRI contrast agents (CAs), such as low molecular weight complexes of gadolinium, are administered. These paramagnetic complexes reduce the longitudinal ($T_1$) and transverse relaxation times ($T_2$*) of the protons of water molecules. Drawbacks of these $T_1$-based contrast agents are their relatively low contrast efficiency, especially at high magnetic field strengths ($B_0$ larger than 1.5 T), and their rapid renal excretion.

An alternative method to generate image contrast utilizes Chemical Exchange-dependent Saturation Transfer (CEST) from selected, magnetically pre-saturated protons to the bulk water molecules. CEST in combination with a paramagnetic chemical shift reagent (ParaCEST) is a promising new MRI method. In this technique, the magnetization of a pool of paramagnetically shifted protons of a CEST contrast agent is selectively saturated by the application of radio frequency (RF) radiation. The transfer of this saturation to bulk water molecules by proton exchange leads to a reduced amount of excitable water protons in the environment of the CEST contrast agent. Thus a decrease of the bulk water signal intensity is observed, which can be used to create contrast enhancement in MRI images.

An approach to obtain a high CEST efficiency is based on utilizing the large number of water molecules of a solution containing a paramagnetic shift reagent (*e.g.* Na[Tm(dotma)($H_2O$)]), wherein"$H_4$dotma" stands for α,α',α,"α'''-tetramethyl-1,4,7,19-tetraacetic acid and dotma represents the respective fourfold deprotonated tetraanionic form of the ligand, to provide a pool of protons that are chemically shifted and that, therefore, can selectively be saturated by an RF pulse. If this system is encapsulated in a carrier, e.g. a liposome, the magnetic saturation can be transferred to the bulk water molecules, at the outside of the carriers, that are not chemically shifted (LipoCEST). The amount of magnetization transfer and hence the extent of contrast enhancement is determined by the rate of the diffusion of water through the shell of the carrier, e.g. a phospholipid membrane, as well as by the amount of water within the carrier.

The optimum water exchange rate is directly correlated with the chemical shift difference between the proton pool inside of the carrier and the bulk water outside of the carrier. The resonance frequency shift that is induced on the water molecules inside the liposomes consists of two main contributions: chemical shift resulting from a direct dipolar interaction between the water molecules and the shift reagent ($\delta_{dip}$), and the resonance frequency shift caused by a bulk magnetic susceptibility effect ($\delta_{bms}$). The overall resonance frequency shift is the sum of these two contributions:

$$\delta = \delta_{dip} + \delta_{bms} \quad (1)$$

$\delta_{bms}$ is zero for spherical particles, but it can be significant for anisotropic particles. The aspherical particles experience a force in a magnetic field, which causes them to align with the magnetic field lines. In the case of liposomes, this effect is further increased, if they bear paramagnetic molecules associated with the phospholipid membrane.

A reference on CEST using aspherical liposomes with paramagnetic molecules associated with the phospholipid membrane is Terreno,E. et al. Angew. Chem. Int. Ed. 46, 966-968 (2007).

LipoCEST contrast agents as referred to above are not sufficiently versatile to be used with analytes other than protons, or with analytes with which the paramagnetic chemical shift reagent poorly interacts.

SUMMARY OF THE INVENTION

It would be advantageous to provide aspherical CEST contrast agents that are more versatile.

In order to address this, in one aspect of the invention, a contrast agent for CEST MRI is provided, wherein the agent comprises a non-spherical carrier that comprises a semipermeable shell, wherein the shell comprises a paramagnetic compound, the shell enclosing a cavity comprising an MR analyte, wherein the semipermeable shell allows

diffusion of the MR analyte and the MR analyte is capable of diffusion through the semipermeable shell, and wherein the cavity does not comprise a paramagnetic shift reagent substantially interacting with the analyte.

In another aspect the MR analyte in the foregoing CEST MRI contrast agent, is of a type not substantially interacting with a paramagnetic chemical shift reagent.

In a further aspect of the invention, the use is presented of a non-spherical CEST MRI contrast agent to conduct MRI on an analyte that does not substantially interact with a paramagnetic chemical shift reagent, wherein the contrast agent comprises a semipermeable shell comprising a paramagnetic compound, the shell enclosing a cavity comprising an amount of the MR analyte and allowing diffusion of the analyte.

In yet another aspect of the invention, the use is presented of a non-spherical CEST MRI contrast agent to obtain MRI contrast enhancement substantially exclusively on the basis of a bulk magnetic susceptibility effect of a pool of an MR analyte, wherein the contrast agent comprises a semipermeable shell comprising a paramagnetic compound, the shell enclosing a cavity comprising the pool of the MR analyte, and allowing diffusion of the analyte.

## DETAILED DESCRIPTION OF THE INVENTION

The invention, in one or more of the above aspects, in fact puts to use the recognition that the paramagnetic chemical shift reagent contained in the cavity of liposomes in lipoCEST contrast agents in fact causes a number of problems.

The chemical shift resulting from a direct dipolar interaction between the molecules of the analyte (typically water) and the shift reagent ($\delta_{dip}$) depends:

a) on a close interaction between these two molecules, and

b) on a fast exchange of the analyte molecule that interacts intimately with the shift reagent, which causes a large chemical shift, and the analyte molecules of the surrounding solution, that are not chemically shifted, so that on average a chemical shift results that is the weighted average of these two values.

This restricts the group of usable analyte molecules to those that directly interact with the internalized shift reagent.

To achieve a sufficiently high resonance frequency shift difference between the inner and the outer compartments of the liposomes, they can be aspherically deformed. This is currently achieved by deforming spherical liposomes in response to a dialysis process against a buffer solution with a higher osmolarity compared to the solution in the cavity of the liposomes. The dialysis causes a net diffusion of water from the cavity of the liposomes into the bulk solution. This reduces the total inner volume of the liposomes. Since the surface area of the liposomes remains constant, the volume reduction forces the liposomes to deform and to assume an aspherical shape, such as a disk shape, a cigar shape, or any other aspherical shape. The bulk magnetic susceptibility effect and the resulting resonance frequency shift ($\delta_{bms}$) scales with the anisotropy of the liposomes. The extent of deformation, in turn, scales with the osmolarity difference between the cavity and the bulk solution. The presence of the shift reagent in the cavity limits the lowest achievable osmolarity in the cavity, which is the concentration of the shift reagent, and hence the maximum achievable resonance frequency shift difference.

The combination of a shift reagent in the cavity of the liposomes and a paramagnetic agent in the phospholipid bilayer requires the incorporation of at least two different paramagnetic compounds in one contrast agent. This increases the number of problem associated with the potential toxicity of these metal complexes that typically contain highly toxic lanthanide ions. These side effects may combine synergistically.

Providing non-spherical CEST carriers as described above, that are not dependent on the action of a paramagnetic chemical shift reagent in its cavity, contributes to several advantages.

Thus, an interaction of the analyte with a shift reagent is not required. The resonance frequency difference between the analyte molecules in the cavity and the surrounding solution is caused predominantly or entirely be the bulk magnetic susceptibility effect. This makes the use of analyte molecules possible that are weak ligands, hence molecules that do not interact strongly with metal ions. Such molecules may include metabolites that are relevant for the detection of certain diseases, such as carbohydrates (e.g. glucose), other metal ions, such as sodium ions, or hyperpolarized gases, such as xenon or helium.

Further, in applying the principle of the invention to carriers that are by nature spherical, in the process of rendering these carriers non-spherical, the osmolarity difference between the cavity of the carrier and the surrounding solution can be maximized to induce the maximum achievable deformation (anisotropy) of the particles in the dialysis step. This effect becomes particularly important in *in vivo* experiments, in which the osmolarity of the surrounding solution is given by the body fluids and cannot be influenced. Thus the deformation of the CEST contrast carrier and hence the resonance frequency shift difference *in vivo* is limited by the number of molecules in the cavity of the carrier. By virtue of a CEST effect determined by an optimized shape (substantial degree of anisotropy), the number of analyte molecules

in the cavity can be minimized in the CEST contrast carriers of the invention.

With the CEST contrast enhancement not based on a paramagnetic chemical shift reagent, only one type of metal complex is required, namely the paramagnetic compound comprised in the shell (such as an amphiphilic compound that is embedded in the bilayer) of a liposome-based CEST contrast carrrier. This contributes to lowering the risk of toxic side effects, in particular this prevents side effects resulting form a combination of different metal complexes in a single agent.

Carriers

The non-spherical carriers used in the present invention are defined with reference to a semipermeable shell enclosing a cavity. The term "semipermeable" is well understood in the art. In general it refers to the property of a shell, such as a membrane, to be selectively permeable, sometimes also denoted partially or differentially permeable. It indicates a structure that basically is closed in the sense that it is a not fully open, and preferably mostly closed, wall (in this case a shell enclosing a cavity), that allows certain molecules or ions to pass through it by diffusion.

In this description, the semipermeability of the shell generally refers to its ability to allow the MR analyte to pass through it by diffusion. Hence, if the combination of analyte (such as water, sodium, noble gas, or small organic molecules) and shell (such as a lipid bilayer) is such that the analyte is capable of passing through the shell by diffusion, the shell is considered to be semipermeable.

Liposomes are generally spherical vesicles comprising a bilayer membrane enclosing a cavity. The bilayer can be made up of at least one phospholipid and may or may not comprise cholesterol. Liposomes can be composed of naturally-derived phospholipids with mixed lipid chains (like egg phosphatidylethanolamine), or of pure surfactant components like DOPE (dioleoylphosphatidylethanolamine). The term liposomes, as used in the description of the invention, includes lipid spheres usually denoted micelles.

A typical example of a semipermeable shell is also found in semipermeable membranes comprising a phospholipid bilayer. A phospholipid bilayer is the most permeable to small, uncharged solutes. Liposomes can be made on the basis of a phospholipid bilayer. Alternatively, the nonspherical carrier used in the present invention can be based on erythrocytes. As another alternative, the carrier used in the present invention can be based on polymersomes or other capsules comprising a polymeric shell.

Liposomes and other potential carriers based on a semipermeable shell enclosing a cavity, will generally be spherical. For use in the invention, such spherical carriers need to be rendered aspherical. E.g. in the case of liposomes, this is done by subjecting the liposomes to a dialysis process against a hypertonic buffer solution, hence a buffer solution with a higher osmolarity compared to the solution at the inside of the liposomes. The dialysis causes a net diffusion of water from the inside of the liposomes to the extraliposomal solution. This reduces the total inner volume of the liposomes. Since the surface area of the liposomes remains constant, the volume reduction forces the liposomes to deform and to assume an aspherical shape, such as a disk shape, a cigar shape, or any other aspherical shape.

This deformation is essentially reversible, since exposure of these so deformed liposomes to an isotonic (with respect to the original, pre-deformation buffer concentration) or hypotonic buffer solution, results in a recovery of their original spherical shape. Therefore, the extent of the induced resonance frequency shift difference depends on the osmolarity of the extraliposomal solution. Therefore, liposomes acccording to the present invention may be used as sensors for the detection of differences of the osmolarity or the pH of the extraliposomal fluid.

Liposomes can be provided with an MR analyte in various ways. Mostly, the desired MR analyte will be encapsulated, i.e. surrounded by a semipermeable shell, during the process of making the liposomes. Alternatively liposomal carriers may be opened reversibly (e.g. in response to osmotic stress similar to cell lysis protocols), filled with an amount of the desired analyte molecules, and re-sealed. In another alternative, pre-formed liposomal carriers may be filled with the desired analyte molecules by passive diffusion over the liposome membrane in a dialysis process.

Liposomes are well known in the art, and are disclosed, e.g., for drug delivery. Types of liposomes available for drug delivery, and methods of making liposomes having a desired agent contained therein, can be applied also for providing liposomes containing an MR analyte.

Intrinsically non-spherical carriers can be based on erythrocytes, viz. by employing erythrocyte ghosts. In order to provide a semipermeable shell that encloses a cavity comprising an MR analyte, erythrocytes are used that have lost most, and preferably all, of their original water-soluble contents. The resulting, MR analyte-containing erythrocytes are more appropriately referred to as erythrocyte ghosts. Thus, particles result in which an MR analyte is contained in a membrane which happens to be the phospholipid bilayer originating from an erythrocyte.

Erythrocytes are reported to be potential biocompatible vectors for bioactive substances, e.g. drugs. A review on this use of erythrocytes is Millan,C.G., et al., "Drug, enzyme and peptide delivery using erythrocytes as carriers." Journal of Controlled Release 95, 27-49 (2004).

Previously described applications include the encapsulation of MRI $T_1$ and $T_2$* contrast agents. The present invention relates to an entirely different type of MRI contrast enhancement, viz. CEST, and to benefits specific to CEST.

By making use of erythrocyte ghosts, the present invention effectively utilizes the erythrocyte membrane as a shell for a CEST contrast agent.

Erythrocytes that can be utilized in the present invention are preferably erythrocytes originating from species that have erythrocytes without a nucleus. Thus, it is preferred to use mammalian erythrocytes, and most preferably human. The latter provides an additional advantage of inherent biocompatibility. Also, it opens up the possibility of using erythrocytes harvested from the same person as will be subjected to MRI using the resulting, MR analyte-loaded erythrocyte ghosts.

The MR analyte-loaded erythrocyte ghosts are obtainable by a process comprising the steps of providing erythrocytes, subjecting the erythrocyte to hypotonic lysis so as to provide an opening in the erythrocyte membrane, subjecting the opened erythrocyte to one or more washing steps so as to substitute a medium being the MR analyte (such as water), or a solution or dispersion of an MR analyte (such as metabolites dispersed or dissolved in water), or any other liquid comprising a desired MR analyte, for at least part of the original water-soluble remove contents of the erythrocyte, and subjecting the resulting MR analyte-loaded erythrocyte ghosts to a closing step under isotonic conditions.

Although, preferably, the washing and filling steps are conducted as a concerted action, it is also possible to conduct the washing steps as separate washing and filling steps, whereby in a first step, or first series of steps, the opened erythrocytes are subjected to a washing step so as to remove the original water-soluble contents (preferably all of the cell cytoplasm), and in a second step, or a second series of steps, the thus emptied erythrocytes are contacted with a liquid as referred to above under such conditions as will allow the liquid to enter the emptied erythrocytes. E.g. by contacting the emptied erythrocytes with an aqueous solution or dispersion. Such contacting can be done, e.g. by incubating the opened erythrocytes in an aqueous solution or dispersion.

The MR analyte

The MR analyte is present in the cavity of the carrier. Its determination, by MR, according to the invention is based on the aforementioned bulk magnetic susceptibility effect. According to this effect, the analyte (generally a pool of analyte molecules or ions) is subject to a resonance frequency shift as a result of the non-spherical carriers' alignment with the magnetic field, as outlined above. When the CEST contrast carrier is subjected to an *in vivo* MRI experiment, in the same way as in more conventional CEST contrast enhancement, the magnetization of the analyte in the non-spherical carrier can be selectively saturated by means of an RF pulse of sufficiently narrow bandwidth. In view of the diffusion of analyte from the cavity of the carrier to the surrounding solution (such as body fluid), said saturation will be transferred to the outside environment of the contrast carrier. Thus, upon conducting magnetic resonance imaging, the direct environment of the carriers will show a decreased signal intensity as compared to the more distant analyte molecules, and this allows to detect the direct environment of the contrast agents due to a decreased signal intensity.

In a desirably simple embodiment, in the context of proton MRI, the MR analyte is water. However, the CEST contrast carriers of the present invention also serve the better use of other analyte molecules or ions, such as sodium, (small) organic molecules, or (hyperpolarized) noble gases such as xenon or helium.

Small molecules, notably small organic molecules, with a molecular weight of less than 500 g/mol and containing less than 40 carbon atoms, preferably containing less than 20 carbon atoms, to the extent they can be provided in conjunction with a semipermeable shell allowing their diffusion, can also be used. These molecules will typically be included in the cavity of the carrier as a solution or dispersion in a liquid medium, such as water, a water-based buffers solution, a cell lysate, or a lipophilic oil, such as one comprising aliphatic oils or perfluoro carbon compounds or combinations thereof.

This may find use, e.g. if the occurrence of metabolites or other molecules that play a role in physiological processes is to be assessed. Such analyte molecules will generally be present as a solution or dispersion in a continuous phase, such as an aqueous solution, in the carrier's cavity. Since the shell allows diffusion of the analyte, the analyte will be able to exchange with the same analyte molecules if and when present in the bulk fluid (such as human body fluid) on which the MRI is conducted. This process is sufficiently specific for the desired analyte molecules on the basis of the frequency ofpre-saturation, the frequency of detection, and the typically relatively high concentration of the desired analyte molecules. It will be apparent to the person skilled in the art that the carrier can be optimized for the exchange performance of the desired molecules, e.g. by tailoring the semipermeability of the shell.

Other MR analytes include ions such as sodium or (hyperpolarized) noble gases. In view of the latter, reference can be made to MRI based on hyperpolarized helium or xenon. A reference for MRI based on hyperpolarized helium is van Beek,E.J., Wild,J.M., Kauczor,H.U., Schreiber,W., Mugler,J.P., de Lange,E.E., Functional MRI of the lung using hyperpolarized 3-helium gas. J Magn Reson Imaging, 20, 540-254 (2004).

The contrast carriers of the invention contribute to an improvement of a promising method of xenon-based MR imaging. A reference to the application of the CEST-contrast concept with hyperpolarized xenon as the analyte, in combination with a biological targeting ligand for molecular imaging is Schröder,L., Lowery,T.J., Hilty,C., Wemmer,D.E. & Pines,A., Molecular Imaging Using a Targeted Magnetic Resonance Hyperpolarized Biosensor. Science 314, 446-449

(2006). This technique has been dubbed HyperCEST (see also Fig. 11). Similar to the conventional "ParaCEST" approach in proton NMR/MRI, only one or a few atoms of the analyte molecules (Xe in HyperCEST, H in ParaCEST) are addressed by the pre-saturation pulse that is applied at the specific CEST resonance frequency, at which only the analyte molecule comprised in the contrast agent absorbs. In H NMR/MRI the sensitivity is increased by the LipoCEST approach, in which a large assembly of H atoms that is enclosed in a nanoparticle (lipososome) is addressed at any time. Similarly in the here proposed LipoHyperCEST approach, a large assembly of Xe atoms that is enclosed in a nanoparticle (lipososome) is addressed at any time in the pre-saturation phase, which in this case induce depolarization of the initially hyperpolarized Xe atoms (see also Fig. 12).

Since Xe is apolar and charge-neutral, hence hydrophobic, it is known to cross a typical lipid bilayer shell as fast as, or faster than the more polar water molecules. Here reference is made to Miller, K.W., Reo,R.V.,Schoot Uiterkamp, A.J.M., Stengle,D.P., Stengle,T.R., Williamson,K.L., Xenon NMR: Chemical Shifts of a General Anesthetic in Common Solvents, Proteins, and Membranes. Proceedings of the National Academy of Science USA 78, 4946-4949 (1981). In this respect, a further advantage of the invention is referred to, as with CEST MRI contrast agents the working of which is based on interaction between a paramagnetic chemical shift reagent and an analyte, the analyte generally needs to be hydrophilic to obtain sufficient interaction by coordination with the paramagnetic chemical shift reagent.

In the HyperCEST approach, the chemical shift difference between Xe (transiently) bound to the contrast agent and the Xe that is not effected by the contrast agent results from a direct dipolar interaction due to coordinative binding to the ligand. In the LipoHyperCEST approach according to this invention, such direct chemical interaction via coordinative binding is not required. Thus the use of noble gases, such as $^{3}He$, for which no suitable ligand is know, as analyte atoms to create CEST-based MR contrast, becomes possible for the first time.

When conducting CEST MRI on the basis of an analyte not naturally occurring in the body (as would be the case with protons in water or in metabolite molecules), such as with a noble gas as xenon, the person subjected to the MRI will be administered a bulk amount of the analyte. In the case of noble gas such as xenon, this will suitably involve nasal, oral, or pulmonal administration, and the MRI technique thus uses are particularly suitable for detection in the gastro-intestinal tract or the respiratory tract.

<u>The paramagnetic compound</u>

The paramagnetic compound can be any compound or complex having paramagnetic properties that can be comprised in the semipermeable shell. Where the semipermeable shell is said to "comprise" the paramagnetic compound, this is a most general reference to any manner in which such a compound can be wholly or partially contained in the shell, or in sufficiently close proximation thereof. Preferably the paramagnetic compound is comprised in the semipermeable shell in such a manner as to be associated with the shell.

The paramagnetic compound preferably is a complex of at least one lanthanide ion selected from the group consisting of $Dy^{3+}$, $Ho^{3+}$, $Er^{3+}$, $Tm^{3+}$, and $Yb^{3+}$ complexed by a multidentate chelating molecule bearing at least one hydrophobic group that comprises at least 6 carbon atoms.

With reference to the use of liposomes, erythrocyte ghosts, polymersomes, or any other carriers having a lipid or polymer shell, the paramagnetic compound preferably is an amphiphilic compound comprising a lanthanide complex (on the more polar side of the amphilic compound), and having an apolar tail that has a tendency to preferably integrate in and align with the lipid bilayer at the carrier's surface based on hydrophobic molecular interactions. These amphilic paramagnetic complexes can be, e.g.:

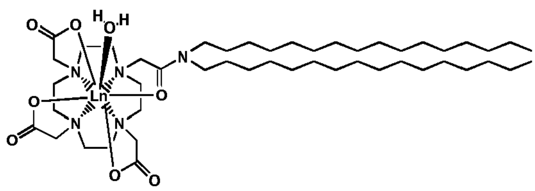

6

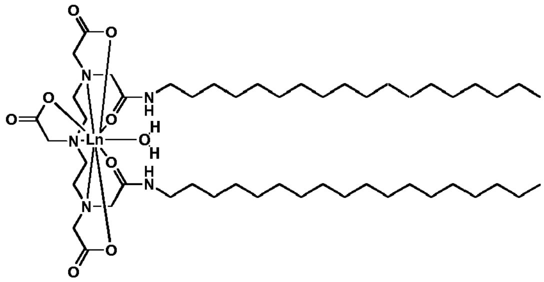

7

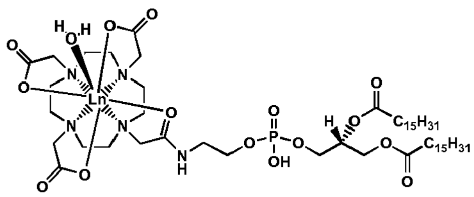

8

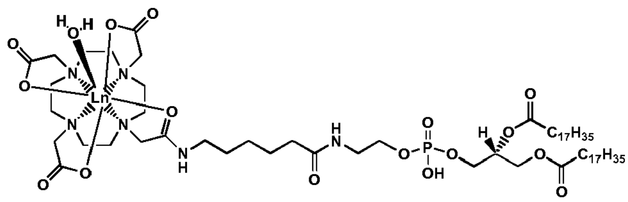

9

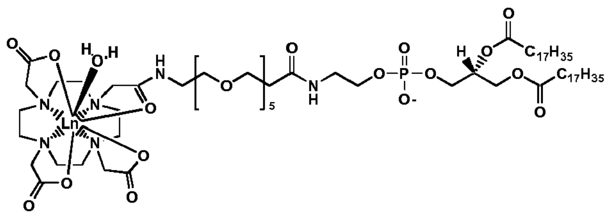

10

The paramagnetic metal complexes do not require a free coordination site for an intimate interaction with the analyte molecules. In fact, a direct interaction with the analyte is disadvantageous, since it may cause undesired $T_1/T_2$* relaxation, which could reduce the amount of built-up magnetic saturation and hence reduce the achievable CEST effect. Therefore these metal complexes are principally different from those used as contact shift reagents in the cavity of the liposomes as discussed hereinafter.

<u>Chemical shift reagents</u>

Although not preferred, it will be apparent from the foregoing, that the carrier, in its cavity, may also comprise a paramagnetic chemical shift reagent. Even in the presence of such an agent at least some of the benefits of the invention can be enjoyed. Thus if, irrespective of some interaction as may exist between the MR analyte and the paramagnetic chemical shift reagent, a substantial portion of the MR analyte does not interact with the shift reagent, even said portion will be subject to CEST contrast enhancement when conducting MRI by virtue of the non-spherical character of the carrier.

This will be the case, e.g., if the MR analyte is a compound such as a metabolite having a weak interaction with the paramagnetic chemical shift reagent.

The paramagnetic chemical shift reagent, if present, will comprise a paramagnetic compound, *i.e.* any compound having paramagnetic properties. Preferably the paramagnetic compound comprises a paramagnetic metal, where the term metal refers to metallic nano- or microparticles, or metal ions, explicitly including metal ions complexed by chelate

ligands. Paramagnetic metals are known to the skilled person, and do not require elucidation here. E.g., early and late transition metals, explicitly including chromium, manganese, iron, as well as lanthanides, such as gadolinium, europium, dysprosium, holmium, erbium, thulium, ytterbium.

The CEST contrast carriers may comprise a $T_1$ or $T_2$* reduction agent. In this respect reference is made to Aime et al., JACS 2007, 129, 2430-2431. In this way an all-in-one concept is realized of $T_1$, $T_2$*, and CEST contrast.

Uses

The nonspherical CEST contrast agents according to the invention can be used in a variety of ways. They can be applied to generate a desired level of MRI contrast in any aqueous environment. Its main use is to generate a local MRI contrast upon *in vivo* application. This can be by introducing the contrast agents, e.g. by injection into the blood or another body fluid of a living being, preferably a human being, and to perform a CEST contrast-enhanced MRI scan of the body, in whole or in part, of said being. The CEST contrast enhancement of bulk water molecules generated, allows the visibility of spots, such as tumors, where the regular body fluid presence is disturbed. Also, the contrast agents of the invention, in their shell can be provided with disease-specific molecular probes, e.g. by having compounds possessing a hydrophobic tail suitable to penetrate into the surface of the carrier (e.g. in the case of a phospholipid surface), wherein the other end of the compounds contains a ligand as desired. This allows the contrast agents to preferentially locate at desired or suspect body sites which then can be made visible by MRI.

The nonspherical contrast agents described according to the invention are particularly versatile. They can be used with non-proton analytes, such as the hyperpolarized xenon embodiment described hereinbefore. They can also be used in the (proton) MRI of molecules such as amines, carbohydrates, or other common metabolic products that may be metabolites linked to a disease or disorder. This enhances the diagnostic use of MRI, the main requirement being that the combination of shell material of the nonspherical CEST carrier, and the analyte, is such that the exchange required for CEST can occur.

Nonspherical CEST contrast carriers of the invention, that do not rely on the presence of a shift reagent in the cavity of the carrier (such as the cavity of a nanoparticle, such as in CEST-active polymer nanocapsules, polymersome etc.) are highly suitable for image-guided drug delivery. Drug delivery from such reagents may be achieved in combination with a formulation of the second compartment, that e.g. may be composed of phospholipids, that induces thermosensitivity. Release of the drug from the cavity of nanoparticle does not coincide with the release of a shift reagent or any other active compound from the cavity. All components that induce a chemical shift are associated with the second compartment (the wall) of the nanoparticle.

In a broad sense, this aspect of the invention can be described with reference to a drug carrier suitable for localized drug delivery, comprising a CEST contrast agent. The suitability of the drug carrier for localized drug delivery can refer to a variety of ways in which a drug carrier loaded with a drug can be triggered to release the drug locally, e.g. by applying a controlled external force or delivering a sufficient amount of energy. This refers, e.g. to thermosensitive drug carriers that can be triggered to locally release a drug by applying local heat. Other methods for localized delivery do not necessarily involve thermosensitive carriers, but carriers that can be triggered to release a drug by a method of activation governed by properties other than thermosensitivity, including but not limited to pH, the presence of a gaseous core and/or layers, sensitive to externally applied ultrasound frequency/wavelength and intensity, and external transcranial energy (e.g. ultrasound) controlled nanomechanical synthetic cells.

A drug carrier in the context of the present invention refers to any material in or on which a bio-active agent can be contained so as to be capable of being released in the body of a subject. Reference is made to the drug carrier comprising a CEST contrast agent. This includes the possibility that the drug carrier as such is adapted for use as a CEST contrast agent.

Suitable carriers include microcarriers and, particularly nanocarriers such as liposomes, polymersomes, nanocapsules and other dosage forms of a size or nature commensurate with a use as a CEST contrast agent.

The drug carrier is to be introduced into the body of a person to be subjected to MRI. This will be e.g. by injection in the blood stream, or by other methods to introduce the carrier into body fluid. If desired, thermosensitive drug carriers can be provided with an enteric coating that does not influence the thermosensitivity, such as cellulose acetate phthalate, or they can be made using a material that serves as an enteric coating and thermosensitive material at the same time, e.g. on the basis of Eudragit®RS/PEG400 blend polymers, see e.g. Fujimori et al., Journal of Controlled Release 102 (1), 2005, 49-57 (PEG stands for polyetyleneglycol).

A drug is a chemical substance used in the treatment, cure, prevention, or diagnosis of a disease or disorder, or used to otherwise enhance physical or mental well-being. The guided delivery foreseen with the present invention will mostly be useful therapeutic agents (i.e. drugs in a strict sense, intended for therapy or prevention of diseases or disorders), but also for agents that are administered for diagnostic purposes. Although other bio-active agents, i.e. those that are not therapeutic or diagnostic, such as functional food ingredients, will not generally be subjected to guided and/or monitored delivery, such could be done using the present invention if desired.

The most optimal use of the invention in the area of drug delivery is attained in the case of targeted therapeutics, *i.e.* drugs that are intended for targeted delivery, as such delivery will by nature benefit most from the monitoring made available by the invention. This pertains, e.g., to agents in the treatment of tumors to be delivered on site, to agents in the treatment or prevention of cardiovascular disorders, such as atherosclerosis in the coronary arteries, or to antithrombotic agents (e.g. for locally resolving blood cloths) or agents that require passing the blood-brain barrier such as neuromodulators as can be used in the treatment of neural conditions such as epilepsy, Alzheimer's disease, Parkinson's disease, or stroke. Benefits from the guidance and monitoring of targeted drug delivery are also applicable to targeted diagnostic agents. Similarly as with targeted therapeutics, here too cancer is an area where site-specific delivery can be of importance.

Bio-active agents suitable for use in the present invention include biologically active agents including therapeutic drugs, endogenous molecules, and pharmacologically active agents, including antibodies; nutritional molecules; cosmetic agents; diagnostic agents; and additional contrast agents for imaging. As used herein, an active agent includes pharmacologically acceptable salts of active agents. Suitable therapeutic agents include, for example, antineoplastics, antitumor agents, antibiotics, antifungals, anti-inflammatory agents, immunosuppressive agents, anti-infective agents, antivirals, anthelminthic, and antiparasitic compounds, including antibodies. Methods of preparing lipophilic drug derivatives that are suitable for nanoparticle or liposome formulation are known in the art (see e.g., US 5,534,499 describing covalent attachment of therapeutic agents to a fatty acid chain of a phospholipid). Drugs in the present invention can also be prodrugs.

The drug may be present in the inner, the outer, or both of the compartments of the carrier, e.g. in the cavity and/or in the shell of a liposome. The distribution of the drug is independent of the distribution of any other agents comprised in the drug carrier, such as a paramagnetic chemical shift reagent or a paramagnetic agent. A combination of drugs may be used and any of these drugs may be present in the inner, the outer, or both of the compartments of the drug carrier, e.g. in the cavity and/or in the shell of a liposome. For use in MRI-guided drug delivery, the invention preferably provides for carriers that are thermosensitive. This means that the physical or chemical state of the carrier is dependent on its temperature.

Any thermosensitive carrier that can package a molecule of interest and that is intact at body temperature (i.e. 37°C) but destroyed at any other, non-body temperature that can be tolerated by a subject may be used. Carriers of the invention include but are not limited to thermosensitive nanoparticles, thermosensitive polymersomes, thermosensitive liposomes, thermosensitive micro- and nanovesicles, and thermosensitive micro- and nanospheres.

Thermosensitive nanovesicles generally have a diameter of up to 100 nm. In the context of this invention, vesicles larger than 100 nm, typically up to 5000 nm, are considered as microvesicles. The word nanovesicle or nanosphere describes any size of vesicle or sphere, explicitly including microvesicles and microspheres according to the above definition. Vesicles, such as liposomal vesicles, typically include a cavity that may contain any substance of interest. In the invention this is preferred, as outlined above.

Thermosensitive nanospheres include but are not limited to spheres that are not smaller than 5 nanometers. Nanospheres typically do not contain a cavity, i.e. in this embodiment of the invention the CEST effect should be realized purely by chemically shifted protons of the paramagnetic chemical shift agent itself that is comprised in the nanosphere.

Thermosensitive polymersomes include but are not limited to any polymer vesicle, including microvesicles and nanovesicles.

Thermosensitive liposomes include but are not limited to any liposome, including those having a prolonged half-life, e.g. pegylated, liposomes.

Thermosensitive liposomes for use in the invention ideally retain their structure at about 37°, i.e. human body temperature, but are destroyed at a higher temperature, preferably only slightly elevated above human body temperature, and preferably also above pyrexic body temperature. Typically about 42°C is a highly useful temperature for thermally guided drug delivery.

The required heat to raise the temperature of the thermosensitive drug carriers so as to promote the destruction of the thermosensitive carriers may be used. Heat can be applied in any physiologically acceptable way, preferably by using a focused energy source capable of inducing highly localized hyperthermia. The energy can be provided through, e.g., microwaves, ultrasound, magnetic induction, infrared or light energy.

Thermosensitive liposomes are known in the art. Liposomes according to the present invention may be prepared by any of a variety of techniques that are known in the art. See, e.g., U.S. Pat. No. 4,235,871; Published PCT applications WO 96/14057; New RRC, Liposomes: A practical approach, IRL Press, Oxford (1990), pages 33-104; Lasic,D.D., Liposomes from physics to applications, Elsevier Science Publishers, Amsterdam, 1993; Liposomes, Marcel Dekker, Inc., New York (1983).

Entrapment of a drug or other bio-active agent within liposomes of the present invention may also be carried out using any conventional method in the art. In preparing liposome compositions of the present invention, stabilizers such as antioxidants and other additives may be used as long as they do not interfere with the purpose of the invention. Examples include co-polymers ofN-isopropylacrylamide (Bioconjug. Chem. 10:412-8 (1999)).

The invention also pertains to a method of performing a CEST MRI scan on a person, wherein CEST contrast agents as described hereinbefore are brought into body fluid of the person and wherein the MRI method includes the application of an RF pulse for which paramagnetically shifted protons in the agent are receptive, so as to saturate the magnetization of said protons, and allowing sufficient time to detect the transfer of said saturation to protons in the outside environment of the contrast agents. Said time typically is of the order of a few seconds.

In one such CEST contrast enhanced MRI scan, typically of from $10^{12}$ M to $10^{-4}$ M (1 pM - 0.1 mM) of CEST contrast agents according to the invention are used.

It is to be understood that the invention is not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, non-limiting Example and the accompanying non-limiting Figures.

In the Examples and the accompanying Figures, the following formulas were used in the calculation of the CEST effect from measured Z spectra of the liposome compositions:

Equation 2:

$$\%CEST = 1 - (M_{on} / M_{off}) \cdot 100\% \qquad (2)$$

$M_{on}$ = intensity after on-resonant pre-saturation
$M_{off}$ = intensity after off-resonant pre-saturation

Equation 3:

$$\%CEST = (M_{off} - M_{on}) / M_{inf} \cdot 100\% \qquad (3)$$

$M_{on}$ = intensity after on-resonant pre-saturation
$M_{off}$ = intensity after off-resonant pre-saturation
$M_{inf}$ = intensity after reference pre-saturation at an "infinitely" distant frequency

BRIEF DESCRIPTION OF THE DRAWINGS

Fig. 1 Magnetic field lines in (a) and around (b) a non-spherical liposome (c) oriented with its longer axis in the direction of a previously uniform, imposed magnetic field [Durrant, C.J., Hertzberg, M.P., Kuchel, P.W. (2003) Magnetic susceptibility: further insights into macroscopic and microscopic fields and the sphere of Lorentz. Concepts Magn Reson Part A 18A:72-95].

Fig. 2 Z Spectra of liposome compositions 1-3

Fig. 3 CEST effect of liposome compositions of examples 1-3, calculated from the Z spectra using Equation 2.

Fig. 4 CEST effect of liposome compositions of examples 1-3, calculated from the Z spectra using Equation 3.

Fig. 5 CEST effect as a function of the applied saturation power for liposome compositions of examples 1-3.

Fig. 6 Z Spectra of liposome composition of example 5 at different field strengths (7T (NMR) and 3T (MRI)).

Fig. 7 CEST effect (calculated from the Z spectrum using Equation 2) of liposome composition of example 5 at different field strengths (7T (NMR) and 3T (MRI)).

Fig. 8 Z Spectra of liposome compositions of examples 6 and 7 (7T (NMR)).

Fig. 9 CEST effect (calculated from the Z spectra using Equation 2) of liposome compositions of examples 6 and 7 (7T (NMR)).

Fig. 10 CEST effect (calculated from the Z spectra using Equation 3) of liposome compositions of examples 5-7 (7T (NMR)).

Fig. 11 Schematic representation of the HyperCEST concept. 1.

Hyperpolarized Xe atoms bind to the Xe-ligand. This induces a shift of the resonance frequency in the MR

experiment, compared to the unbound Xe atoms. 2) RF radiation is applied selectively at the resonance frequency of the bound Xe atoms, which depolarizes them. 3) Depolarized Xe atoms quickly exchange with unbound hyperpolarized Xe atoms. In this way depolarization of a large fraction of the surrounding Xe atoms is achieved. Depolarized Xe atoms give a much smaller signal in the MR experiment, thus a negative contrast enhancement is achieved. A probe for molecular imaging is obtained, if the Xe-ligand is linked to a bio-ligand.

Fig. 12 Schematic representation of the LipoHyperCEST concept. In contrast to the HyperCEST experiment (a), in the LipoHyperCEST experiment (b), according to this invention, a much larger number of Xe atoms is encapsulated in the cage at any point in time, which allows for a much higher sensitivity of the molecular imaging probe.

## Examples

Liposomes were prepared from a phospholipid composition as specified below. The phospolipid composition was dissolved in a mixture of methanol and chloroform (1:3) and the solvents were completely removed under reduced pressure. The resulting thin lipid film was hydrated in a buffer solution (20 mM HEPES (4-(2-hydroxyethyl)-1-piperazine ethane sulfonic acid), pH 7.40). The resulting solution was extruded through 200 nm membranes (8 times) and extensively dialyzed against a hypertonic buffer solution (20 mM HEPES, pH 7.4, 300 mM NaCl, three times overnight at RT) to yield asperical liposomes. The liposomes were studied on a 7 T (300 MHz) NMR spectrometer and partially on a 3 T human MRI scanner for their CEST properties. The structural formulas of the compounds used in the Examples, and the full names thereof, are given below.

*Example 1*

Lipid composition: POPC (55 mol%), PEG (5 mol%), cholesterol (20 mol%), Tm-dtpa-bsa (20 mol%).

*Example 2*

Lipid composition: POPC (55 mol%), PEG (5 mol%), cholesterol (20 mol%), Dy-dtpa-bsa (20 mol%).

*Example 3*

Lipid composition: POPC (55 mol%), PEG (5 mol%), DSPC (10 mol%), cholesterol (20 mol%), Tm-dtpa-bsa (10 mol%).

*Example 4*

Lipid composition: POPC (60 mol%), Cholesterol (20 mol%), Tm-dtpa-bsa (18 mol%), Yb-dotam-dspe (2 mol%).

*Example 5*

Lipid composition: DSPC (55 mol%), POPC (20 mol%), PEG (5 mol%), Tm-dtpa-bsa (20 mol%).

*Example 6*

The sample of Example 5, which in the final step was dialyzed against a buffer containing an NaCl concentration of 300 mM, was further dialyzed two times overnight against a buffer containing an NaCl concentration of only 100 mM (20 mM HEPES, pH 7.4).

*Example 7*

Lipid composition: DSPC (55 mol%), POPC (20 mol%), PEG (5 mol%), Dy-dtpa-bsa (20 mol%).

Table 1

Summary of the CEST results of the liposome compositions 1-7 at 7T

| Example | Amphiphile (mol%)[a] | Chem. Shift (ppm) | %CEST (Equation 3) |
|---|---|---|---|
| 1 | Tm(20) | -13 | 34 |
| 2 | Dy(20) | +13 | 25 |
| 3 | Tm(10) | -7 | 20 |

(continued)

Summary of the CEST results of the liposome compositions 1-7 at 7T

| Example | Amphiphile (mol%)[a] | Chem. Shift (ppm) | %CEST (Equation 3) |
|---|---|---|---|
| 4 | Tm(18) | +10 | 32 |
| 5 | Tm(20) | -18 | 60 |
| 6[b] | Tm(20) | -8 | 50 |
| 7 | Dy(20) | +18 | 50 |

[a] Ln(%) = Ln-dtpa-bsa (%)
[b] Dialysis buffer containing 100 mM sodium chloride and 20 mM HEPEs, pH 7.40.

**Structural formulas of the used molecules**

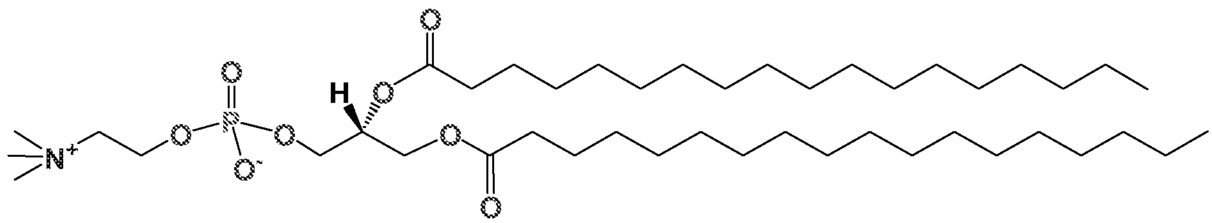

1,2-Distearoyl-*sn*-glycero-3-phosphocholine, **DSPC,** 18:0-18:0 PC

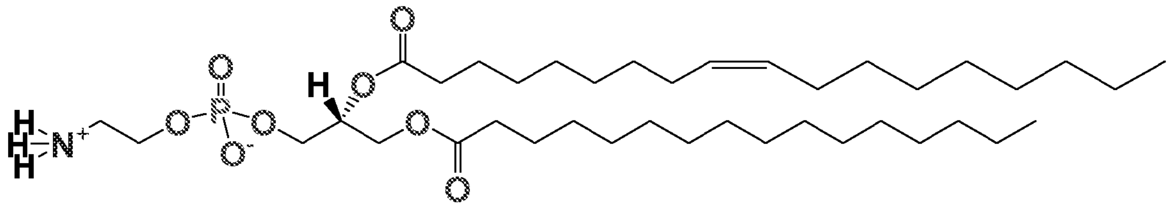

1

-Palmitoyl-2-oleoyl-*sn*-glycero-3-phosphocholine, **POPC**, 16:0-18:1 PC

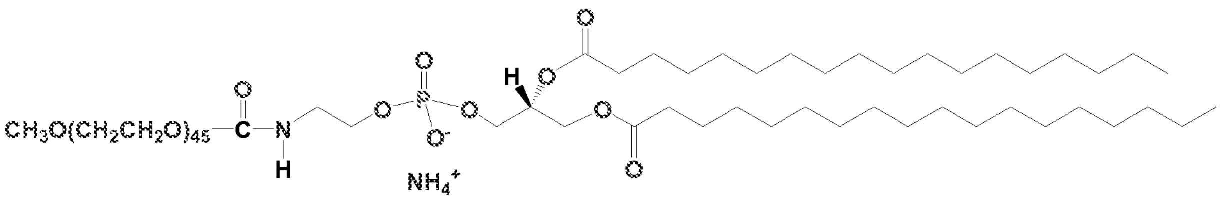

1,2-Distearoyl-*sn*-glycero-3-phosphoethanolamine-N-[methoxy(polyethylene glycol)-2000] (ammonium Salt), DSPE-PEG, DSPE-PEG2000Me, 18:0-18:0 PE PEG2, **PEG**

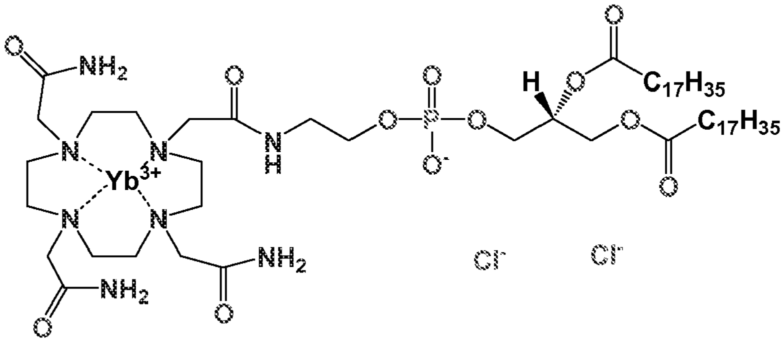

Ytterbium-dotam-1,2-Distearoyl-*sn*-glycero-3-phosphoethanolamine), **Yb-dotam-dspe,** Yb-dotam-18:0-18:0 PE

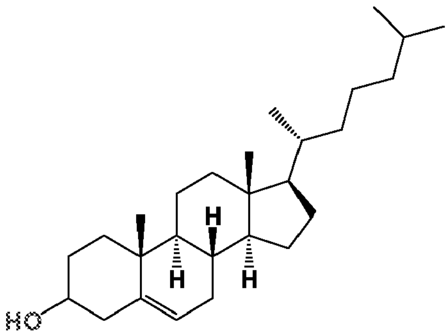

Cholesterol

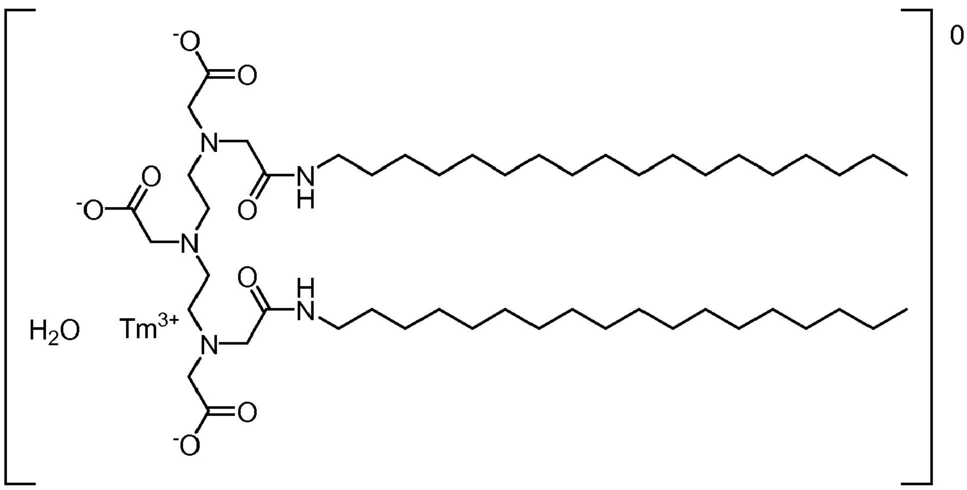

Thullium complex of diethylenetriaminepentaacetic acid bis(stearylamide), thullium aqua diethylenetriamine-1,7- bis((N-stearyl)carbamoylmethly)-1,4,7-triacetate, [Tm(dtpa-bsa)($H_2O$)], **Tm-dtpa-bsa**

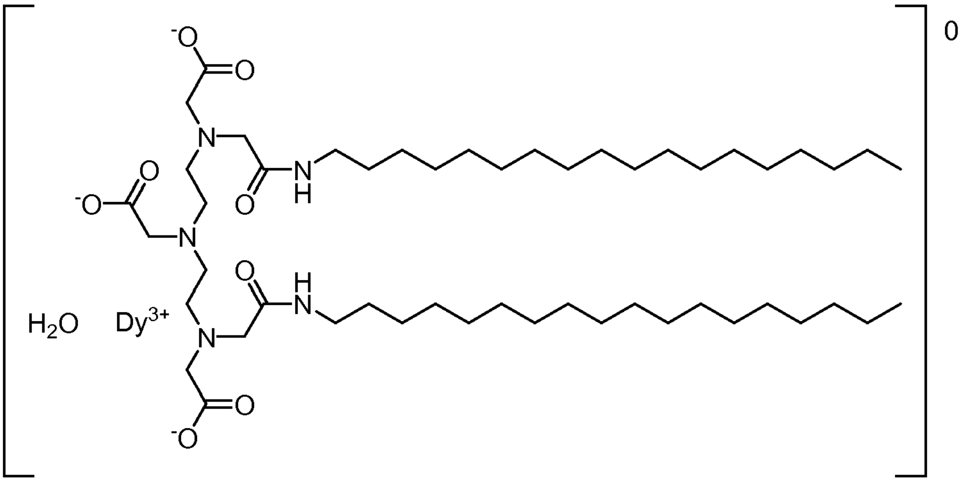

Dysprosium complex of diethylenetriaminepentaacetic acid bis(stearylamide), dysprosium aqua diethylenetriamine-1,7-bis((N-stearyl)carbamoylmethyl)-1,4,7-triacetate, [Dy(dtpa-bsa)($H_2O$)], **Dy-dtpa-bsa**

## Claims

1. A contrast agent for Magnetic Resonance Imaging (MRI) based on Chemical Exchange-dependent Saturation Transfer (CEST), the agent comprising a non-spherical carrier comprising a semipermeable shell, wherein the shell comprises a paramagnetic compound, the shell enclosing a cavity comprising an MR analyte, wherein the semipermeable shell allows diffusion of the MR analyte and the MR analyte is capable of diffusion through the semipermeable shell, and wherein the cavity does not comprise a paramagnetic shift reagent substantially interacting with the analyte.

2. A contrast agent for CEST MRI according to claim 1, wherein the MR analyte is of a type not substantially interacting with a paramagnetic chemical shift reagent.

3. A contrast agent for CEST MRI according to claim 1 or 2, wherein the cavity does not substantially comprise a paramagnetic shift reagent.

4. A contrast agent for CEST MRI according to claim 2, wherein the analyte is selected from the group consisting of water, sodium, small organic molecules, and noble gases.

5. A contrast agent according to claim 4, wherein the analyte is hyperpolarized xenon or hyperpolarized helium.

6. A contrast agent according to any one of the preceding claims, wherein the paramagnetic compound is a complex of at least one lanthanide ion selected from the group consisting of $Dy^{3+}$, $Ho^{3+}$, $Er^{3+}$, $Tm^{3+}$, and $Yb^{3+}$ complexed by a multidentate chelating molecule bearing at least one hydrophobic group that comprises at least 6 carbon atoms.

7. A contrast agent according to any one of the preceding claims, wherein the shell comprises a lipid bilayer.

8. A contrast agent according to claim 7, the carrier being selected from the group consisting of liposomes, erythrocyte ghosts, polymersomes, and capsules comprising a polymer shell.

9. A contrast agent according to any one of the preceding claims, the agent comprising a drug, and the carrier being adapted to allow release of the drug through the application of energy.

10. A contrast agent according to claim 9, wherein the carrier is a thermosensitive liposome.

11. A contrast agent according to any one of the preceding claims, comprising a ligand for targeted binding exposed on the outer surface of the carrier.

12. A contrast agent according to claim 11, wherein the ligand comprises a hydrophobic tail, the tail penetrating into a lipid bilayer shell of the carrier.

13. A contrast agent according to claim 11 or 12, wherein the ligand is a disease-specific molecular probe.

14. A method of performing a CEST MRI scan on a person, wherein CEST contrast agents according to any one of claims 1-13 are brought into body fluid of the person and wherein the MRI method includes the application of an RF pulse for which paramagnetically shifted analyte atoms in the agent are receptive, so as to saturate or depolarize the magnetization of said analyte atoms, and allowing sufficient time to detect the transfer of said saturation to the pool of analyte atoms in the outside environment of the contrast agents.

15. A method according to claim 14, wherein the analyte atoms comprise a hyperpolarized noble gas, the person being administered a bulk amount of the noble gas.

16. A method according to claim 15 wherein the bulk amount of the noble gas is administered into the respiratory tract, the method being used in the detection or analysis of pulmonary tumors.

Figure 1

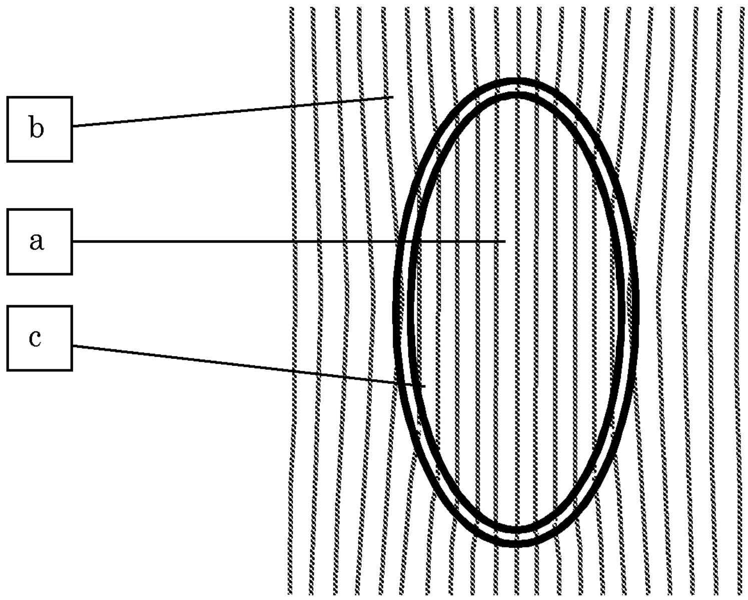

Figure 2

**1**

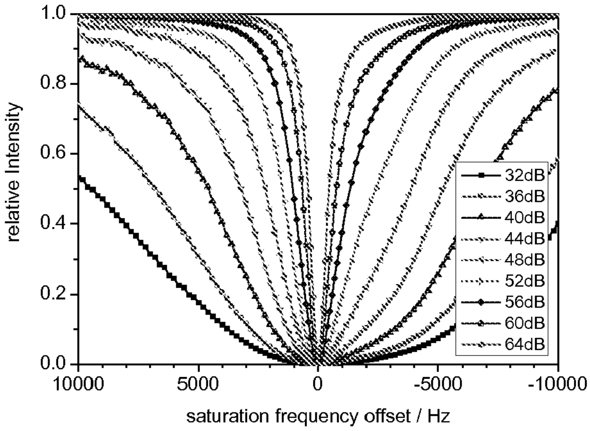
1.0
0.8
0.6
0.4
0.2
0.0
relative Intensity
10000
5000
0
-5000
-10000
saturation frequency offset / Hz
32dB
36dB
40dB
44dB
48dB
52dB
56dB
60dB
64dB

**2**

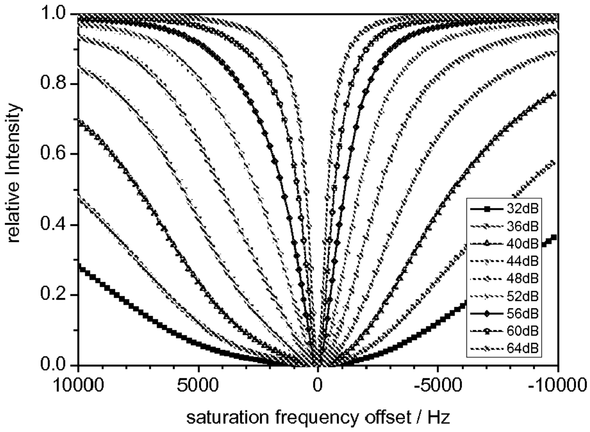
1.0
0.8
0.6
0.4
0.2
0.0
relative Intensity
10000
5000
0
-5000
-10000
saturation frequency offset / Hz
32dB
36dB
40dB
44dB
48dB
52dB
56dB
60dB
64dB

**3**

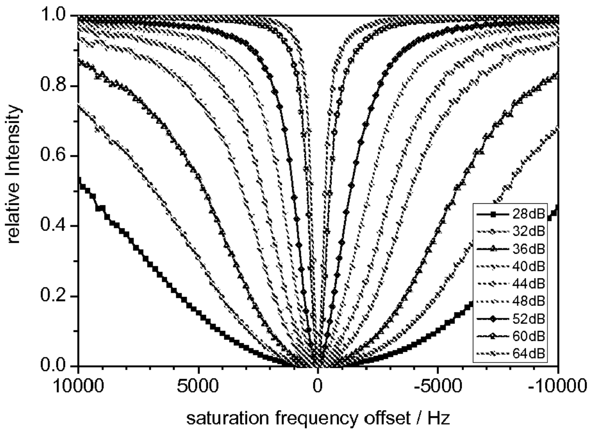
1.0
0.8
0.6
0.4
0.2
0.0
relative Intensity
10000
5000
0
-5000
-10000
saturation frequency offset / Hz
28dB
32dB
36dB
40dB
44dB
48dB
52dB
60dB
64dB

Figure 3

1

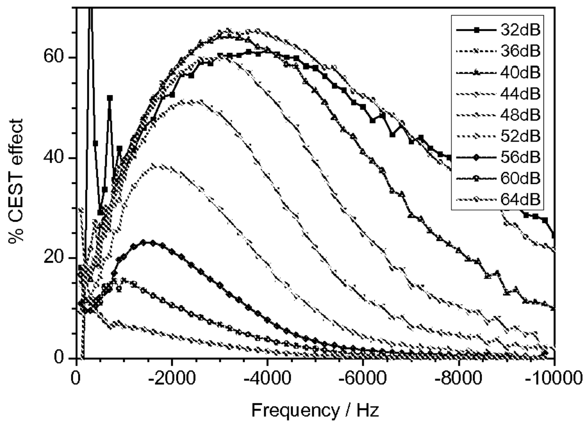
32dB
36dB
40dB
44dB
48dB
52dB
56dB
60dB
64dB
% CEST effect
0
20
40
60
0
-2000
-4000
-6000
-8000
-10000
Frequency / Hz

2

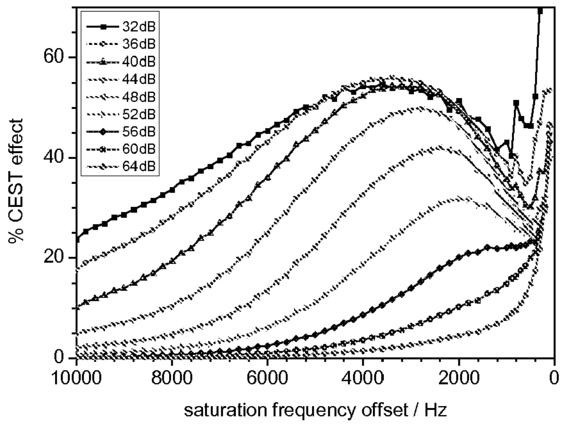
32dB
36dB
40dB
44dB
48dB
52dB
56dB
60dB
64dB
% CEST effect
0
20
40
60
10000
8000
6000
4000
2000
0
saturation frequency offset / Hz

3

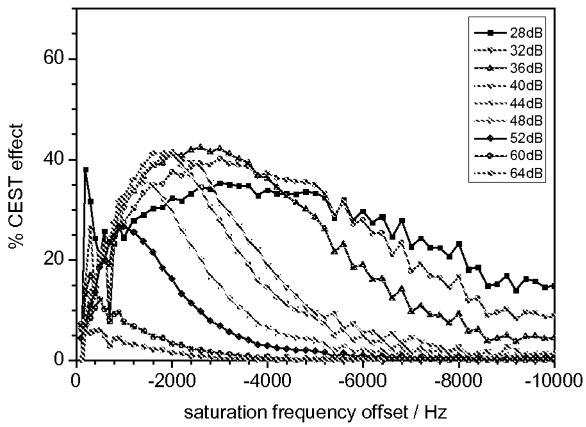
28dB
32dB
36dB
40dB
44dB
48dB
52dB
60dB
64dB
% CEST effect
0
20
40
60
0
-2000
-4000
-6000
-8000
-10000
saturation frequency offset / Hz

Figure 4

1

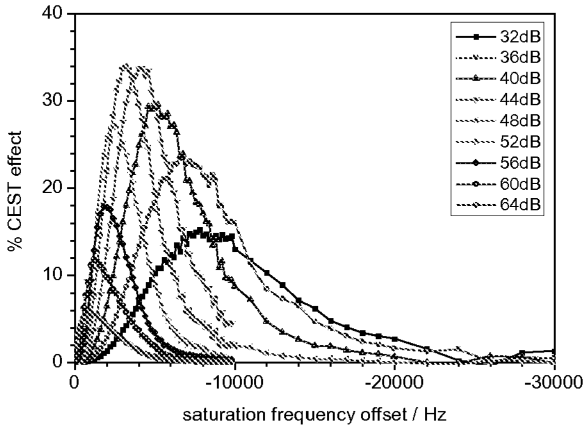
40
30
20
10
0
% CEST effect
0
-10000
-20000
-30000
saturation frequency offset / Hz
32dB
36dB
40dB
44dB
48dB
52dB
56dB
60dB
64dB

2

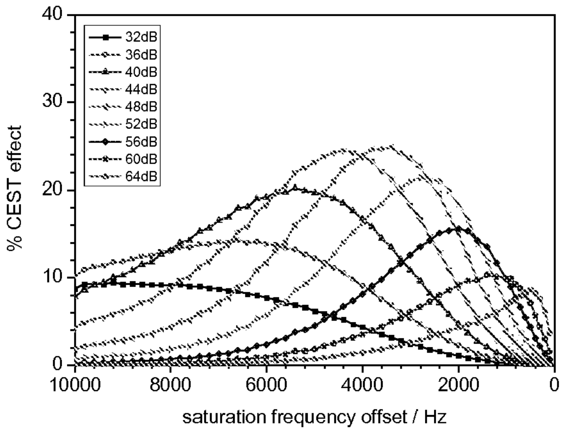
40
30
20
10
0
% CEST effect
10000
8000
6000
4000
2000
0
saturation frequency offset / Hz
32dB
36dB
40dB
44dB
48dB
52dB
56dB
60dB
64dB

3

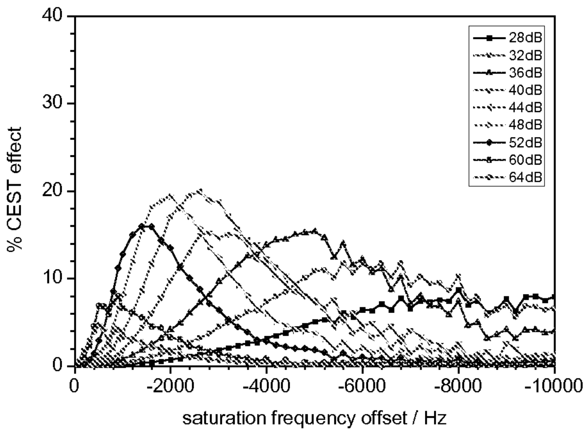
40
30
20
10
0
% CEST effect
0
-2000
-4000
-6000
-8000
-10000
saturation frequency offset / Hz
28dB
32dB
36dB
40dB
44dB
48dB
52dB
60dB
64dB

Figure 5

1

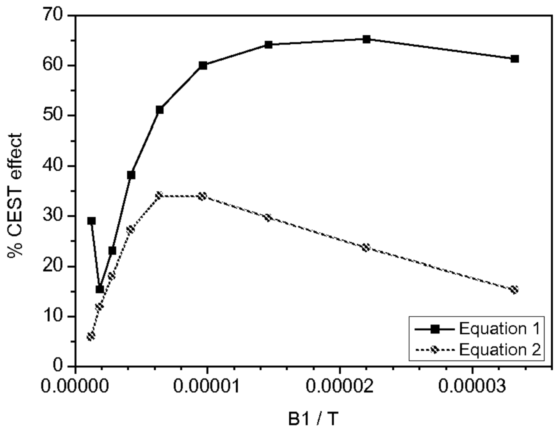
% CEST effect
B1 / T
Equation 1
Equation 2
0
10
20
30
40
50
60
70
0.00000
0.00001
0.00002
0.00003

2

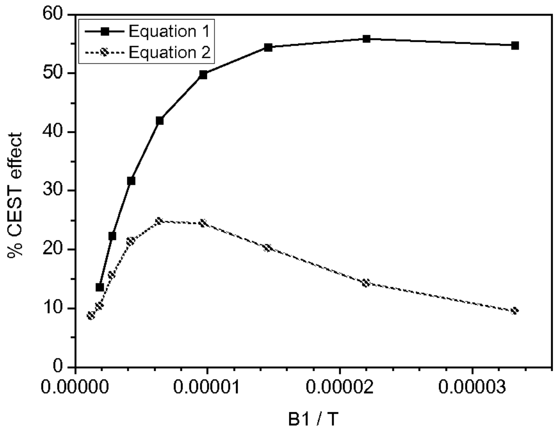
% CEST effect
B1 / T
Equation 1
Equation 2
0
10
20
30
40
50
60
0.00000
0.00001
0.00002
0.00003

3

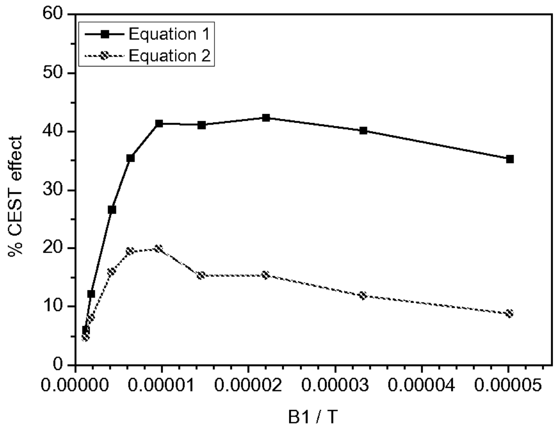
% CEST effect
B1 / T
Equation 1
Equation 2
0
10
20
30
40
50
60
0.00000
0.00001
0.00002
0.00003
0.00004
0.00005

Figure 6

**5 (7T)**

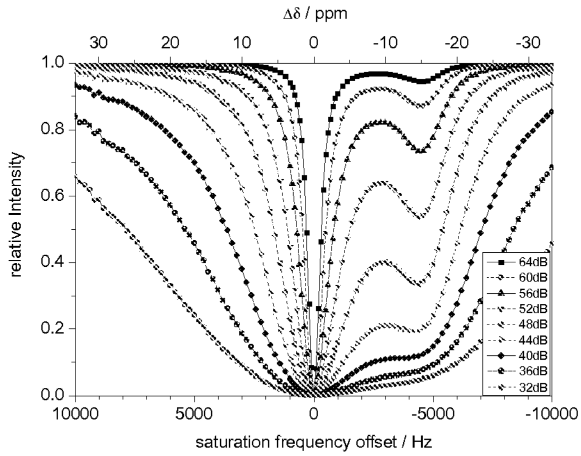


**5 (3T)**

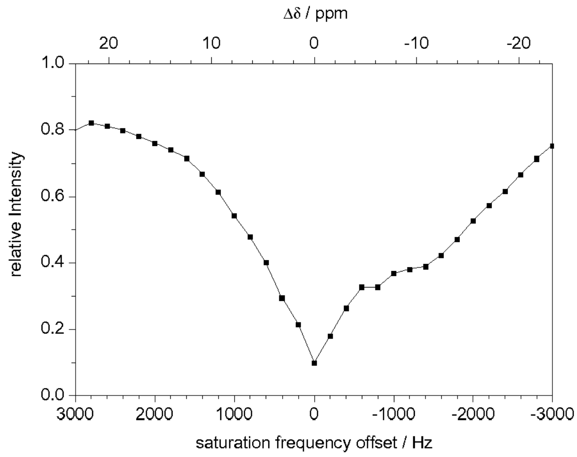

Figure 7

**5 (7T)**

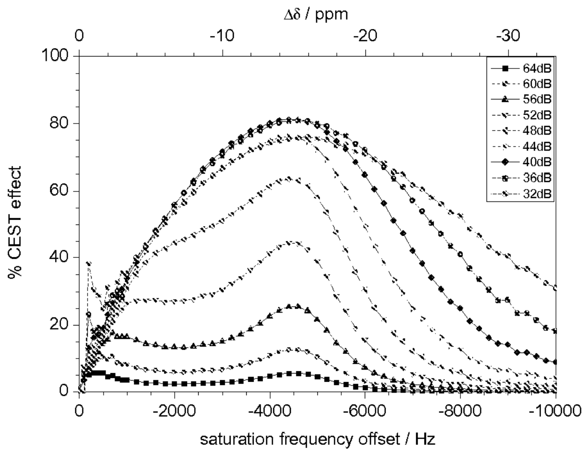


**5 (3T)**

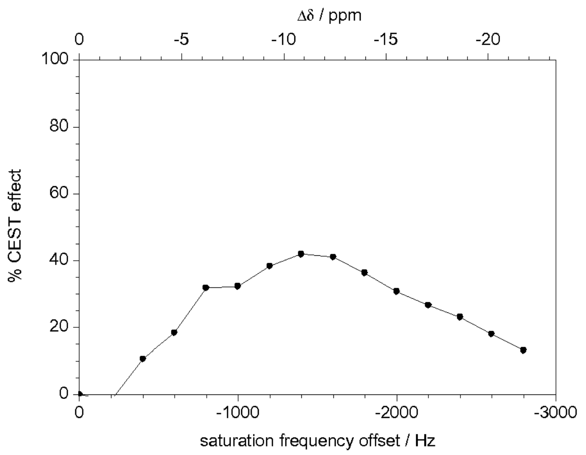

Figure 8

**6 (7T)**

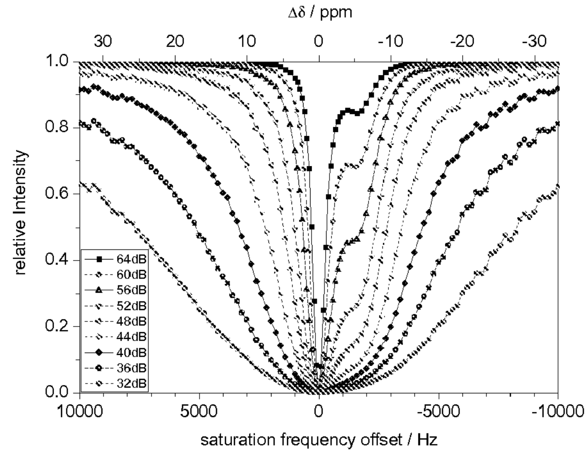


**7 (7T)**

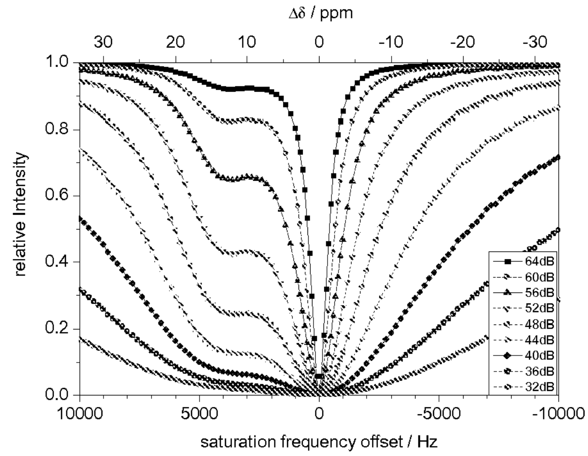

Figure 9

**6 (7T)**

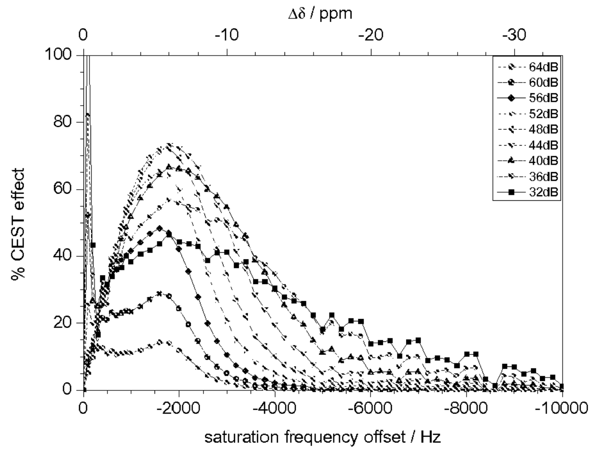


**7 (7T)**

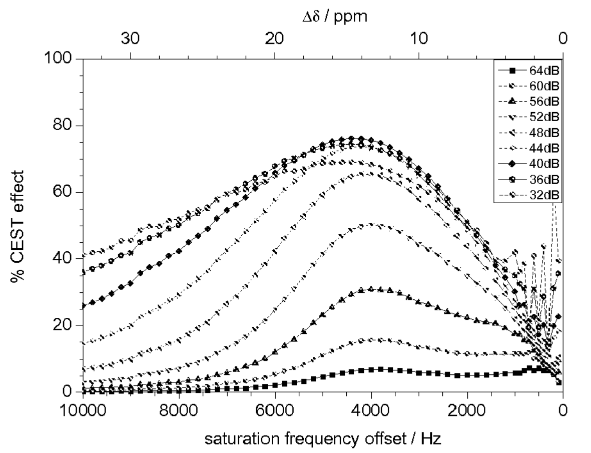

Figure 10

**5 (7T)**

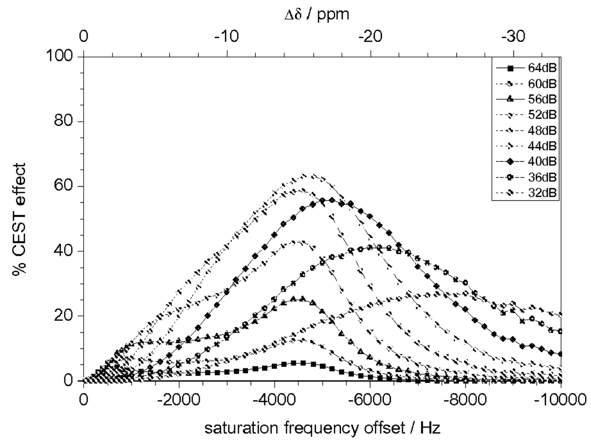


**6 (7T)**

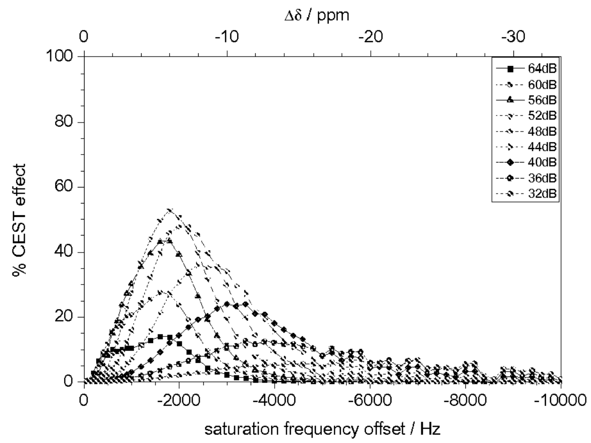


**7 (7T)**

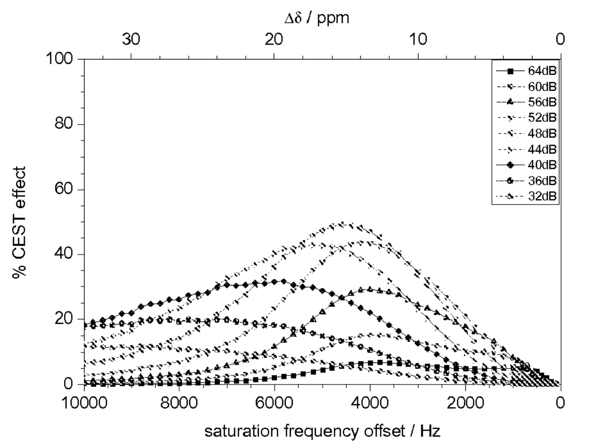

Figure 11

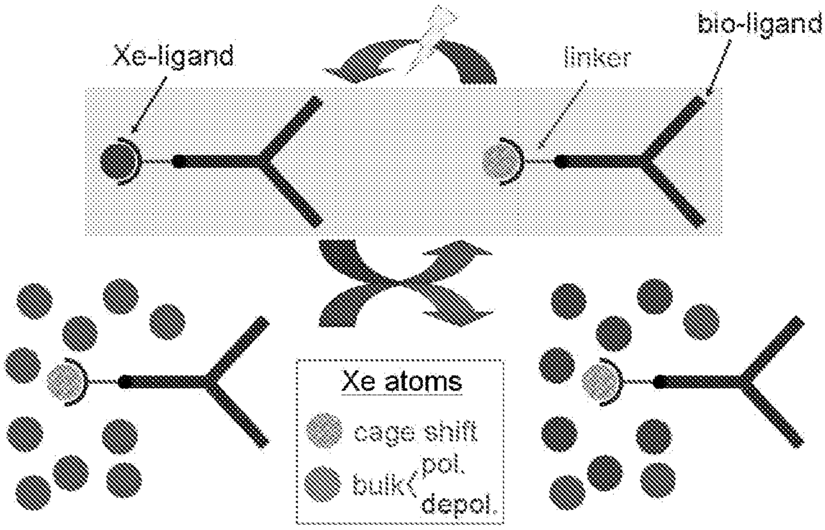


Figure 12

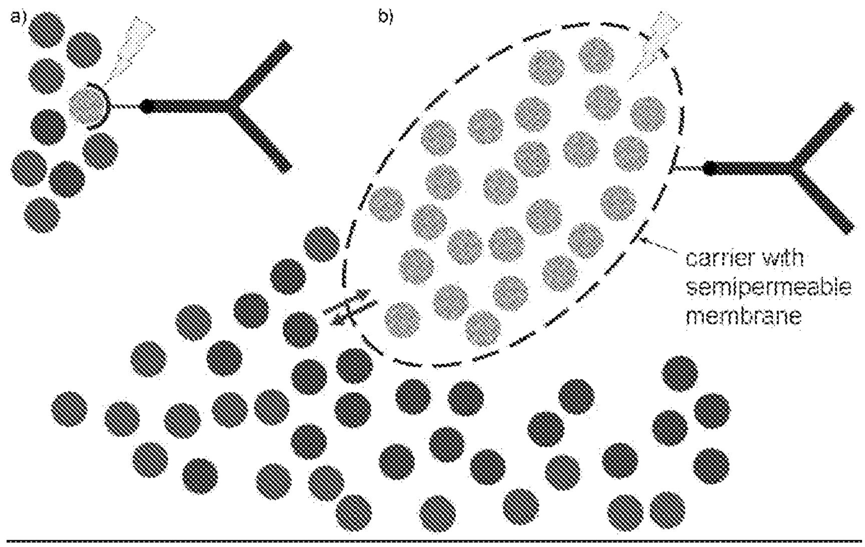

European Patent Office

## PARTIAL EUROPEAN SEARCH REPORT

which under Rule 63 of the European Patent Convention shall be considered, for the purposes of subsequent proceedings, as the European search report

Application Number
EP 07 12 1809

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | TERRENO E ET AL: "From spherical to osmotically shrunken paramagnetic liposomes: an improved generation of LIPOCEST MRI agents with highly shifted water protons" ANGEWANDTE CHEMIE. INTERNATIONAL EDITION, WILEY VCH VERLAG, WEINHEIM, DE, vol. 46, no. 6, 29 January 2007 (2007-01-29), pages 966-968, XP002470079 ISSN: 1433-7851 | 1-4,6-8 | INV. A61K49/18 |
| Y | * page 966, column 2, paragraph 2 - paragraph 3 * * page 967, column 1, paragraph 2 - page 967, column 2, paragraph 2 * * compound 1 * * page 966, column 1, paragraph 1 * ----- -/-- | 5,9-16 | |

TECHNICAL FIELDS SEARCHED (IPC)

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| The Hague | 17 April 2008 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 07 12 1809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X<br><br><br><br><br><br><br><br>Y | TERRENO E. ET AL.: "Highly shifted LIPOCEST agents based on the encapsulation of neutral polynuclear paramagnetic shift reagents" CHEMICAL COMMUNICATION, vol. 8, no. 5, 26 November 2007 (2007-11-26), pages 600-602, XP002476682<br>* page 601, column 2, paragraph 3 *<br>* compounds 1-3 *<br>----- | 1-4,6-8<br><br><br><br><br><br><br><br>5,9-16 | |
| D,Y | SCHRÖDER L. ET AL.: "Molecular imaging using a targeted magnetic resonance hyperpolarized biosensor" SCIENCE, vol. 314, 20 October 2006 (2006-10-20), pages 446-449, XP002476683<br>* the whole document *<br>----- | 5,15,16 | TECHNICAL FIELDS SEARCHED (IPC) |
| A | ANDRESEN T L ET AL: "Advanced strategies in liposomal cancer therapy: Problems and prospects of active and tumor specific drug release" PROGRESS IN LIPID RESEARCH, PERGAMON PRESS, PARIS, FR, vol. 44, no. 1, January 2005 (2005-01), pages 68-97, XP004769683 ISSN: 0163-7827<br>----- | 9-14 | |
| A | VENKATESH A. K. ET AL.: "Hyperpolarized 129Xe MRI using gas-filled liposomes" ACADEMIC RADIOLOGY, vol. 9, no. suppl.1, 2002, pages S270-S274, XP008090368<br>* page S270, column 2, paragraph 2 *<br>* page S273, column 1, paragraph 2 *<br>-----<br>-/-- | | |

3

EPO FORM 1503 03.82 (P04C10)

European Patent Office

# PARTIAL EUROPEAN SEARCH REPORT

Application Number
EP 07 12 1809

| Category | Citation of document with indication, where appropriate, of relevant passages | Relevant to claim | CLASSIFICATION OF THE APPLICATION (IPC) |
|---|---|---|---|
| A | VENKATESH A K ET AL: "EVALUATION OF CARRIER AGENTS FOR HYPERPOLARIZED XENON MRI" NMR IN BIOMEDICINE, WILEY, LONDON, GB, vol. 13, no. 4, June 2000 (2000-06), pages 245-252, XP008069213 ISSN: 0952-3480 * page 252, column 1, paragraph 2 - paragraph 3 * ----- | | |
| A | WO 2006/032705 A (GUERBET SA [FR]; PORT MARC [FR]) 30 March 2006 (2006-03-30) ----- | | |
| A | WO 2006/114738 A (KONINKL PHILIPS ELECTRONICS NV [NL]; WILLARD NICOLAAS P [NL]; WEGH REN) 2 November 2006 (2006-11-02) ----- | | TECHNICAL FIELDS SEARCHED (IPC) |

3

EPO FORM 1503 03.82 (P04C10)

European Patent Office

INCOMPLETE SEARCH SHEET C

Application Number
EP 07 12 1809

Although claims 14-16 are directed to a method of treatment of the human/animal body (Article 53(c) EPC), the search has been carried out and based on the alleged effects of the compound/composition.

-----

## ANNEX TO THE EUROPEAN SEARCH REPORT ON EUROPEAN PATENT APPLICATION NO. EP 07 12 1809

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2008

| Patent document cited in search report | Publication date | Patent family member(s) | Publication date |
|---|---|---|---|
| WO 2006032705 A | 30-03-2006 | EP 1793868 A2 | 13-06-2007 |
| | | US 2007292354 A1 | 20-12-2007 |
| WO 2006114738 A | 02-11-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5534499 A
- US 4235871 A
- WO 9614057 A


### Non-patent literature cited in the description


- **TERRENO,E. et al.** *Angew. Chem. Int. Ed.,* 2007, vol. 46, 966-968
- **MILLAN,C.G. et al.** Drug, enzyme and peptide delivery using erythrocytes as carriers. *Journal of Controlled Release,* 2004, vol. 95, 27-49
- **VAN BEEK,E.J. ; WILD,J.M. ; KAUCZOR,H.U. ; SCHREIBER,W. ; MUGLER,J.P. ; DE LANGE,E.E.** Functional MRI of the lung using hyperpolarized 3-helium gas. *J Magn Reson Imaging,* 2004, vol. 20, 540-254
- **SCHRÖDER,L. ; LOWERY,T.J. ; HILTY,C. ; WEMMER,D.E. ; PINES,A.** Molecular Imaging Using a Targeted Magnetic Resonance Hyperpolarized Biosensor. *Science,* 2006, vol. 314, 446-449
- **MILLER, K.W. ; REO,R.V. ; SCHOOT UITERKAMP,A.J.M. ; STENGLE,D.P. ; STENGLE, T.R. ; WILLIAMSON,K.L. ; XENON NMR.** Chemical Shifts of a General Anesthetic in Common Solvents, Proteins, and Membranes. *Proceedings of the National Academy of Science USA,* 1981, vol. 78, 4946-4949
- **AIME et al.** *JACS,* 2007, vol. 129, 2430-2431
- **FUJIMORI et al.** *Journal of Controlled Release,* 2005, vol. 102 (1), 49-57
- New RRC, Liposomes: A practical approach. IRL Press, 1990, 33-104
- **LASIC,D.D.** Liposomes from physics to applications. Elsevier Science Publishers, 1993
- Liposomes. Marcel Dekker, Inc, 1983
- *Bioconjug. Chem.,* 1999, vol. 10, 412-8
- **DURRANT, C.J. ; HERTZBERG, M.P. ; KUCHEL, P.W.** Magnetic susceptibility: further insights into macroscopic and microscopic fields and the sphere of Lorentz. *Concepts Magn Reson Part A,* 2003, vol. 18A, 72-95